# EUROPÄISCHE PATENTANMELDUNG

(11) **EP 3 251 654 A1**
(43) Veröffentlichungstag der Anmeldung: **06.12.2017**
(21) Anmeldenummer: 16171904.2
(22) Anmeldetag: 30.05.2016
(51) Int. Cl.: A61K 8/39, A61K 8/63, A61Q 90/00, A61K 31/08, A61K 31/19

(54) **NICHTIONISCHE TENSIDE ZUR REDUKTION VON FETTGEWEBE**

(71) Anmelder: CHEMISCHE FABRIK KREUSSLER & CO. GMBH, 65203 Wiesbaden (DE)
(72) Erfinder: Travers, Stephan., 65187 Wiesbaden (DE); Gliem, Petra., 65232 Taunusstein (DE); Lotter, Matthias., 65201 Wiesbaden (DE); Otto, Joachim., 65366 Geisenheim-Stephanshausen (DE)
(74) Vertreter: dompatent von Kreisler Selting Werner - Partnerschaft von Patent- und Rechtsanwälten mbB

(57) **Zusammenfassung**

Die vorliegende Erfindung betrifft eine kosmetische Zusammensetzung zu Reduktion von Fettgewebe, die Verwendung dieser Zusammensetzung sowie ein Verfahren zur Reduktion von ungewolltem Fettgewebe.

## Beschreibung

Die vorliegende Erfindung betrifft eine kosmetische Zusammensetzung zu Reduktion von Fettgewebe, die Verwendung dieser Zusammensetzung sowie ein Verfahren zur Reduktion von unerwünschtem Fettgewebe.

Zunehmende Fettleibigkeit in der Bevölkerung stellt ein deutliches Problem insbesondere der Industrieländer dar. Dies hat Auswirkungen auf die Kosten der Gesundheitssysteme. Gleichzeitig wird ein Schönheitsideal propargiert, welches einen sehr schlanken Körper als Optimum darstellt. Dabei werden insbesondere lokale Fettpölsterchen, wie beispielsweise im Bereich der Wangenknochen oder des Kinns bereits als unattraktiv angesehen.

Um nun überschüssiges Fett zu entfernen sind unterschiedliche Möglichkeiten bekannt. Eine verbesserte Form der Fettabsaugung ist beispielsweise in EP 1 733 692 A1 offenbart.

Um die Risiken einer Operation zu umgehen werden zunehmend Injektionen eingesetzt, die entsprechende Fettpölsterchen entfernen sollen. So offenbart beispielsweise WO 02/060410 A2 die Verwendung des Signalstoffes TNF-α (Tumornekrose-Faktor-α) in einer entsprechenden Injektion. Die Kombination aus einem Chelatbildner für Eisen, einem osmotisch wirksamen Mittel, Aminosäuren und Vitaminen ist in WO 2013/093947 A1 offenbart.

Aufgabe der vorliegenden Erfindung ist es eine Zusammensetzung zur Reduktion von Fettgewebe bereit zu stellen, die langanhaltend und wirksam ist, den Stand der Technik bereichert und eine Alternative zu den bekannten Zusammensetzungen bietet. Zudem soll es zu möglichst geringen Nebenwirkungen, wie beispielsweise allergischen Reaktionen oder ähnlichem kommen.

Gelöst werden die Aufgaben durch Zusammensetzungen gemäß dem Hauptanspruch. Vorteilhafte Ausführungsformen sind in den Unteransprüchen genannt.

Weiterhin umfasst die Erfindung die Verwendung der Zusammensetzung sowie ein Verfahren zur Reduktion von Fettgewebe, wie in den nebengeordneten Ansprüchen dargestellt.

Überarschenderweise und für den Fachmann nicht vorhersehbar hat sich gezeigt, dass nichtionische Tenside geeignet sind, Fettgewebe von Menschen zu entfernen. Eine solche Entfernung erfolgt durch Verabreichung einer wässrigen Lösung der Tenside in das Fettgewebe, so dass eine kosmetische Zusammensetzung gemäß der vorliegenden Erfindung wenigstens ein nichtionisches Tensid als aktive Substanz sowie Wasser als Lösungsmittel aufweist. Das Wasser ist dabei für entsprechende Anwendungen geeignet und ist insbesondere Wasser für Injektionszwecke.

Das nichtionische Tensid ist insbesondere ausgewählt aus Polidocanol und Dihydroxycholansäure. Dabei sind auch Salze der Tenside erfindungsgemäß hiervon umfasst. Dihydroxycholansäure ist eine Desoxycholsäure und gehört zu den sekundären Gallensäuren. Bekannt ist deren Wirkung zum Auflösen von Gallensteinen. Überraschenderweise hat sich nun gezeigt, dass 3α,12α-Dihydroxycholansäure (Dihydroxycholansäure, Desoxycholsäure) ebenfalls in der Lage ist Fettzellen zu zerstören.

Besonders bevorzugt ist das nichtionische Tensid Polidocanol. Polidocanol ist eine Kurzbezeichnung für Polyethylenglykol(9)monododecylether, welches der folgenden Formel entspricht:

C₁₂H₂₅-(O-CH₂-CH₂)ₙ-OH.

Üblicherweise nimmt n einen Durchschnittswert von 9 an. Das durchschnittliche Molekulargewicht liegt bei etwa 600 g/mol. Polidocanol ist auch bekannt unter Nonaoxytylen-Monodecylether, PEG-9, Laurylether, Polyethylenglykol 450, Laurylether Polyoxyethylene (9).

Es wird angenommen, dass die aktive Substanz, also das nichtionische Tensid, eine Abnahme der Oberflächenspannung der Zellmembrane der Fettzellen bewirkt. Hierdurch kann Flüssigkeit aus der extrazellulären Matrix (ECM) in die Fettzelle eindringen. Dies führt zu einer Zellschwellung, wodurch ein osmotischer Austausch stattfindet. Die Fettzelle gibt so ihren Inhalt, insbesondere Fettsäuren, wie beispielsweise Triglyzeride, und Zytoplasma frei. Die bisher in den Fettzellen gespeicherten Fettsäuren können dann über das Lymphsystem abgeführt und aus dem Körper ausgeschieden werden.

Polidocanol weist vorzugweise ein mittleres Molekulargewicht im Bereich von 500 bis 700, insbesondere von 570 bis 600 und/oder ein Cloud-Point in Wasser von 80°C bis 90°C auf. Es hat sich gezeigt, dass in diesem Fall die Zusammensetzung besonders gut verträglich ist und wenige Nebenwirkungen auftreten. Auch die Wirksamkeit ist in diesem Fall gut.

Sowohl Polidocanol als auch Dihydroxycholansäure sind bereits als Medikamente zugelassen, so dass das Risiko hinsichtlich unerwünschter Wirkungen im menschlichen Körper so gering wie möglich ist. Negative Langzeitwirkungen sind aus der bisherigen Anwendung im Wesentlichen nicht bekannt. Überraschenderweise hat sich nun eine neue Verwendung, nämlich die lokale Fettentfernung, gezeigt, so dass die vorliegende Erfindung ebenfalls die Verwendung von nichtionischen Tensiden und insbesondere von Polidocanol beziehungsweise Dihydroxycholansäure zur lokalen Entfernung von Fettgewebe betrifft.

Die erfindungsgemäße kosmetische Zusammensetzung kann ein nichtionisches Tensid als aktive Substanz enthalten. Es ist auch möglich, dass sie 2, 3 oder mehrere unterschiedliche nichtionische Tenside enthält. Vorzugsweise enthält sie Polidocanol und/oder Dihydroxycholansäure. Besonders bevorzugt ist die aktive Substanz Polidocanol. Der Anteil an nichtionischen Tensid in der Zusammensetzung beträgt vorzugsweise 0,1 Gew.% bis 20 Gew.%, insbesondere 0,3 Gew.% bis 15 Gew.% oder bis 10 Gew.%, insbesondere 0,5 Gew.% bis 8 Gew.% oder bis 5 Gew.%, besonders bevorzugt 0,8 Gew.% bis 3 Gew.% oder bis 1 Gew.%.

Neben der aktiven Substanz kann die wässrige Zusammensetzung weitere Hilfsstoffe aufweisen. Hilfsstoffe sind dabei solche Verbindungen, die die Wirkung gegebenfalls unterstützen oder die Stabilität der Zusammensetzung verbessern. Eine eigene Wirkung hinsichtlich der Fettentfernung kann diesen jedoch nicht zugeschrieben werden. Sie können jedoch eine eigene Wirkung aufweisen, wie beispielsweise eine Verringerung der Irritation des Gewebes, welches das zu entfernende Fettgewebe umgibt. Entsprechende Hilfsstoffe sind erfindungsgemäß insbesondere ausgewählt aus Vitamine, Aminosäuren, Verzögerungsmittel, Lösungsvermittlern sowie Mischung dieser.

Vitamine und Aminosäuren werden zugegeben, um insbesondere das dem Fettgewebe umliegende Gewebe mit Nährstoffen zu versorgen, so dass eine möglichst geringe Nebenwirkung erreicht wird. Zudem stellt sich so nach der Behandlung ein attraktives Hautbild dar.

Lösungsvermittler im Sinne der vorliegenden Erfindung sind solche Stoffe, die das nichtionische Tensid im Lösungsmittel, also Wasser, stabil lösen, sodass es über einen längeren Zeitraum hinweg, von wenigstens einer Woche oder länger, insbesondere von zwei Wochen oder länger, insbesondere von vier Wochen oder länger, vorzugsweise von drei Monaten oder länger, insbesondere von sechs Monaten oder länger, bei Raumtemperatur stabil gelagert werden kann. Ein Lösungsvermittler sorgt zudem für eine homogene Verteilung der aktiven Substanz im Lösungsmittel. Gleichzeitig ist der Lösungsvermittler ein solcher Stoff, der mit dem Fettgewebe nicht wesentlich in Wechselwirkung tritt, also die Wirkung des aktiven Stoffs nicht beeinflusst. Gleichzeitig treten keine weiteren Nebenwirkungen auf.

Bevorzugt ist der Lösungsvermittler vorliegend ausgewählt aus Alkoholen, insbesondere aus einwertigen und/oder mehrwertigen Alkoholen. Mehrwertige Alkohole im Sinne der vorliegenden Erfindungen sind vorzugsweise zwei- und/ oder dreiwertige Alkohole. Besonders bevorzugt als Lösungsvermittler sind entsprechende zwei- und/oder dreiwertige Alkohole mit weniger als 10 Kohlenstoffatomen, insbesondere mit 2 bis 5 Kohlenstoffatomen (C-Atomen). Neben Kohlenstoff und Wasserstoff weisen die Alkohole zusätzlich zu der Hydroxygruppe keine weiteren Atome auf. Besonders bevorzugt ist der Lösungsvermittler ein Diol, also ein zweiwertiger Alkohol mit 2 bis 5 Kohlenstoffatomen. Diese sorgen für eine homogene, gleichmäßige Verteilung sowie eine Stabilisierung der aktiven Substanz im Lösungsmittel und gleichzeitig führt es zu keinerlei unerwünschten Nebenwirkung.

Ein Verzögerungsmittel im Sinne der vorliegenden Erfindung ist eine solche Substanz, die nach Verabreichung der Zusammensetzung in das Fettgewebe dafür sorgt, dass die aktive Substanz gleichmäßig über einen längeren Zeitraum hinweg freigesetzt wird. Hierdurch wird ein gleichmäßiger Wirkspiegel über eine gewünschte Dauer hinweg ermöglicht, wodurch eine besonders gute Fettentfernung ermöglicht wird. Das Verzögerungsmittel kann beispielsweise eine Mikrokapsel sein. Diese kann beispielsweise in Form einer Kern-Hülle-Kapsel vorliegen. Im Kern enthält eine solche Kapsel die Aktivsubstanz. Die Hülle besteht dabei aus einem Material, welches im Fettgewebe löslich ist, sich jedoch in der wässrigen Lösung selbst nicht auflöst. Hierdurch kann es zu einer verzögernden Freisetzung der aktiven Substanz erst am Ort, an dem sie wirken soll, kommen. Gleichzeitig ist ein Transport zum gewünschten Ort sichergestellt, ohne dass es zu einer großflächigeren Verteilung der Aktivsubstanz und damit zu einer verringerten Konzentration führen kann. Mikrokapseln können auch Speckles sein. Hierbei handelt es sich um solche Partikel, in welchem eine Matrix vorliegt. In diese Matrix ist dann die Aktivsubstanz eingebunden. Sowohl die Matrix von Speckles als auch die Höhle von Kern-Hülle-Kapseln können aus an sich bekannten Materialien bestehen, wie beispielsweise und bevorzugt Polylactiden, Polyglycoliden, Polymilchsäuren, Polyglykolsäuren, Polyanhydriden, Polyorthoesthern, Polyetheresthern, Polycaprolactonen, Polyestheramiden, Polycarbonaten, Polycyanoacrylaten, Polyurethanen, Polyacrylaten und Mischungen oder Copolymeren dieser. Verzögerungsmittel können auch Alginate sein. Bevorzugt ist das Verzögerungsmittel eine Mikrokapsel, welche Alginate und/oder Poly(lactid-co-glycolid) als Matrix beziehungsweise Hülle-Material aufweist.

Um nun eine konstante Freisetzung der aktiven Substanz über einen langen Zeitraum zu ermöglichen, kann die erfindungsgemäße Zusammensetzung die aktive Substanz beispielsweise unmittelbar gelöst aufweisen. Weiterhin kann sie Mikrokapseln, die das Verzögerungsmittel sowie die Aktivsubstanz aufweisen, enthalten. Wird die kosmetische Zusammensetzung in das Fettgewebe injiziert, ist zum einen eine unmittelbare Wirkung sichergestellt. Durch das Verzögerungsmittel wird dann ein langfristiger Austrag über einen Zeitraum von wenigstens drei Tagen, vorzugsweise von fünf Tagen oder länger sichergestellt. Der Zeitraum, über welchen das nichtionische Tensid freigesetzt wird, kann durch die Wahl des Verzögerungsmittels individuell eingestellt werden.

Die vorliegende Erfindung betrifft weiterhin die Verwendung einer kosmetischen Zusammensetzung wie zuvor beschrieben zur Reduktion von Fettgewebe mittels Injektionslipolyse. Injektionslipolyse ist dabei die Verabreichung der Zusammensetzung mittels Injektion für die Behandlung lokaler Fettdepots. Lokale Fettdepots können sich dabei am ganzen Körper befinden, insbesondere werden hierdurch Doppelkinn, Hängebäckchen, Oberarme, Achseln, Bauch, Hüften, Reiterhosen, Knie, Fesseln, Fettansammlungen an Po, Brust und an der Innenseite der Oberschenkel reduziert. Auch bei Pseudo-Gynäkomastie oder beim sogenannten Stiernacken ist eine Verwendung der erfindungsgemäßen Zusammensetzung möglich.

In einer weiteren Ausführungsform betrifft die vorliegende Erfindung ein Verfahren zur Reduktion von Fettgewebe. Dieses umfasst die Verabreichung einer kosmetischen Zusammensetzung wie zuvor beschrieben in das zu behandelnde Areal. Das zu behandelnde Areal ist dabei das Gebiet eines Körpers, insbesondere eines menschlichen Körpers, in welchem sich eine lokale Fettansammlung befindet. Dies kann dabei alle Regionen des menschlichen Körpers betreffen. Insbesondere umfasst sind dabei Kinn, Wangen, Oberarme, Achseln, Bauch, Hüften, Knie, Fesseln, Po, Brust und/oder Oberschenkel. Dabei erfolgt die Verabreichung vorzugsweise mittels Injektion. Insbesondere erfolgt die Injektion in das Unterhautfettgewebe des zu behandelnden Areals. Somit kann die aktive Substanz unmittelbar ihre Wirkung entfalten. Durch das in einer bevorzugten Ausführungsform erhaltene Verzögerungsmittel kann insbesondere in solchen Arealen, in denen größere Ansammlungen von Fettgeweben vorherrschen, größere Mengen der Zusammensetzung eingebracht werden und diese dort länger wirken.

Um eine Sicherstellung der Wirkung zu erhalten, ist eine Wiederholung der Verabreichung empfehlenswert. Diese sollte jedoch in einem gewissen zeitlichen Abstand erfolgen, damit der Körper ausreichend Zeit hat, die freigesetzten Fettsäuren aus dem Körper auszuscheiden. Weiterhin sollte sich das umliegende Gewebe des zu behandelnden Areals erholen können.

Auch wenn die aktiven Substanzen, Lösungsmittel und Hilfsstoffe einer ausführlichen Prüfung unterzogen werden, so kann es dennoch zu unerwünschten Reaktionen im Körper kommen. Damit diese möglichst gering gehalten werden, ist ein zeitlicher Abstand von wenigstens einer Woche einzuhalten. Insbesondere erfolgt die Verabreichung in einem zeitlichen Abstand von 7 bis 10 Tagen.

Weist die Zusammensetzung ein besonders wirksames Verzögerungsmittel auf, welches die Freisetzung über eine Zeitraum von 3 Tagen oder mehr, insbesondere von bis zu 7 Tagen ermöglicht, erfolgt die Verabreichung in einem zeitlichen Abstand von 18 bis 30 Tagen oder sogar einem längeren Abstand.

## Patentansprüche

1. Kosmetische Zusammensetzung zur Reduktion von Fettgewebe umfassend
a) wenigstens ein nichtionisches Tensid als aktive Substanz und
b) Wasser als Lösungsmittel.

2. Zusammensetzung nach Anspruch 1, **dadurch gekennzeichnet, dass** das nichtionische Tensid ausgewählt ist aus Polidocanol und Dihydroxycholansäure und insbesondere Polidocanol ist.

3. Zusammensetzung nach Anspruch 2, **dadurch gekennzeichnet, dass** das Polidocanol ein mittleres Molekulargewicht im Bereich von 500 bis 700, insbesondere von 570 bis 600, und/oder einen Cloud-Point in Wasser von 80 °C bis 90 °C aufweist.

4. Zusammensetzung nach einem der Ansprüche 1 bis 3, **dadurch gekennzeichnet, dass** der Anteil an nichtionischem Tensid 0,1 Gew.-% bis 20 Gew.-%, insbesondere 0,3 Gew.-% bis 10 Gew.-% beträgt.

5. Zusammensetzung nach einem der Ansprüche 1 bis 4, weiterhin umfassend einen oder mehrere Hilfsstoffe ausgewählt aus Vitaminen, Aminosäuren Verzögerungsmitteln, Lösungsvermittlern und Mischungen dieser.

6. Zusammensetzung nach Anspruch 5, **dadurch gekennzeichnet, dass** der Lösungsvermittler ausgewählt ist aus einwertigen Alkoholen und/oder mehrwertigen Alkoholen, insbesondere aus zwei- und/oder dreiwertigen Alkoholen mit weniger als 10 C-Atomen, insbesondere mit 2 bis 5 C-Atomen.

7. Zusammensetzung nach Anspruch 5, **dadurch gekennzeichnet, dass** das Verzögerungsmittel eine Mikrokapsel ist, welche als Kern-Hülle-Kapsel oder als Speckles vorliegt.

8. Verwendung einer kosmetischen Zusammensetzung nach einem der Ansprüche 1 bis 7 zur Reduktion von Fettgewebe mittels Injektionslipolyse.

9. Verfahren zur Reduktion von Fettgewebe umfassend die Verabreichung einer kosmetischen Zusammensetzung nach einem der Ansprüche 1 bis 7 in das zu behandelnde Areal.

10. Verfahren nach Anspruch 9, **dadurch gekennzeichnet, dass** die Verabreichung mittels Injektion insbesondre ins Unterhautfettgewebe erfolgt.

11. Verfahren nach Anspruch 9 oder 10, **dadurch gekennzeichnet, dass** die Verabreichung wiederholt erfolgt, insbesondere in einem zeitlichen Abstand von 7 bis 10 Tagen.

12. Verfahren nach Anspruch 9 oder 10, **dadurch gekennzeichnet, dass** die Verabreichung wiederholt erfolgt, insbesondere in einem zeitlichen Abstand von 18 bis 30 Tagen oder mehr.
